Europäisches Patentamt

European Patent Office

Office européen des brevets

(11) Publication number: **0 314 355**
**A2**

## EUROPEAN PATENT APPLICATION

(21) Application number: **88309675.2**

(22) Date of filing: **14.10.88**

(51) Int. Cl.⁴: **C07C 69/14 , C07C 67/54 , C07C 19/07 , C07C 17/38**

(30) Priority: **24.10.87 GB 8724971**

(43) Date of publication of application:
**03.05.89 Bulletin 89/18**

(84) Designated Contracting States:
**AT BE DE ES FR GB IT LU NL SE**

(71) Applicant: **BP Chemicals Limited
Belgrave House 76 Buckingham Palace Road
London, SW1W 0SU(GB)**

(72) Inventor: **Gulliver, David Jeffrey
BP Chemicals Limited Salt End
Hedon Hull HU12 8DS(GB)**

(74) Representative: **Denbigh, Keith Warwick et al
BP INTERNATIONAL LIMITED Patents
Division Chertsey Road
Sunbury-on-Thames Middlesex, TW16
7LN(GB)**

(54) Separation process.

(57) A process for separating a mixture comprising methyl iodide, methyl acetate and optionally acetone is provided. The process comprises an extractive countercurrent distillation using acetic acid as the extractant in which an upward flow of the mixture in vapour flow is contacted with a downward flow of liquid acetic acid. The process is suitably carried out in a distillation column having between 20 and 60 plates.

EP 0 314 355 A2

## SEPARATION PROCESS

The present invention relates to a distillation process for separating methyl iodide and methyl acetate and for separating methyl acetate from a mixture of methyl acetate,methyl iodide and acetone.

In recent years carbonylation processes, in particular rhodium catalysed carbonylation processes, have become industrially important. The most important of such processes, e.g. the production of acetic acid by carbonylation of methanol (GB 1,233,121) and the production of acetic acid and acetic anhydride by the carbonylation of methanol/methyl acetate/water mixtures (e.g. EP 87870) involve the use of substantial amounts of methyl iodide.

A problem arises when such processes give rise to streams comprising either methyl acetate and methyl iodide or methyl acetate, methyl iodide and acetone. The problem is that, because such components form respectively a binary or ternary azeotrope, the components cannot be separated by simple distillation. It is therefore necessary to resort to expensive and often sophisticated procedures to recover the components. This can be major drawback on commercial plant since, as methyl iodide is both expensive and toxic, it is necessary to recover and reuse as much of it is possible.

A distillation process is now provided which allows these troublesome azeotropes to be broken. Using the process of the present invention therefore the components of the azeotrope can be recovered essentially pure by a simple method.

According to the present invention there is provided a process for separating a mixture comprising methyl iodide and methyl acetate which process comprises

(a) introducing the mixture of methyl iodide and methyl acetate into the lower half of a distillation zone.

(b) introducing acetic acid into the upper half of the distillation zone.

(c) removing overhead an overhead fraction having a methyl iodide content greater than the methyl iodide content of a methyl acetate/methyl iodide azeotrope.
and

(d) removing at the bottom of the distillation zone a bottom fraction comprising methyl acetate and acetic acid.

The process of the present invention breaks the methyl acetate/methyl iodide azeotrope by contacting the azeotrope, which is generated in the distillation zone, with a countercurrent of acetic acid. As the distillation zone is operated so that the head of the column is maintained at the boiling point of methyl iodide (ca 43°C at atmospheric pressure) and the boiling point of acetic acid is 118°C under similar conditions, the process operates by contacting an upward flow of the azeotrope vapour with the downwards flow of liquid acetic acid. The acetic acid therefore acts as a selective extractant for the methyl acetate.

A similar effect occurs if the azeotrope comprises methyl acetate, methyl iodide and acetone with the acetic acid selectively extracting the methyl acetate and the acetone. Accordingly in an embodiment of the present invention there is provided a process for separating a mixture comprising methyl iodide, methyl acetate and acetone which process comprises

(a) introducing the mixture of methyl iodide, methyl acetate and acetone into the lower half of a distillation zone.

(b) introducing acetic acid into the upper half of the distillation zone.

(c) removing overhead on overhead fraction having a methyl iodide greater than the methyl iodide content of a methyl acetate/methyl iodide/acetone azeotrope
and

(d) removing at the bottom of the distillation zone a bottom fraction comprising methyl acetate, acetone and acetic acid.

The process of the present invention is suitably carried out using a conventional distillation column mounted upon a kettle. In such an embodiment the distillation column acts as the distillation zone. Using such a configuration is is preferable to employ a distillation column having between 20 and 60 plates and to feed the methyl acetate and methyl iodide into the bottom of the distillation column preferably between a third and a half way up the column. In order to minimise carryover of acetic acid in the overhead fraction it is preferable that the acetic acid be introduced into the distillation column at a point below the top but within the top third.

It is preferred that a reflux ratio in the range 2:1 to 10:1 by volume is employed. Most preferably the reflux ratio is in the range 5:1 to 8:1. The reflux : acetic acid and reflux : acetic acid heads ratio are preferably in the ranges 5:1 to 15:1 and 1:0.8 to 1:2.5 respectively.

The present invention is now illustrated with reference to the following Examples.

## Example 1

The distillation was carried out continuously in a 45 plate glass Oldershaw column mounted upon a kettle. A feed comprising ( % by wt)

74.6% methyl iodide
16.2% methyl acetate
0.05% acetic acid
2.6% acetone

was fed to the column at plate 10 at a rate of 2053 ghr$^{-1}$. Acetic acid (441 ghr$^{-1}$) was fed, together with the reflux, to the head of column. The head of the column was maintained at 43°C. At steady-state, the overhead and bottom fractions had the following composition ( % by weight - determined by glc).

## Overhead

93% methyl iodide
0.98% methyl acetate
5.6% acetic acid
0.13% acetone
Productivity 1723 gh$^{-1}$

## Bottom

4.25% methyl iodide
38.8% methyl acetate
43.5% acetic acid
5.77% acetone
Productivity 811 gh$^{-1}$

A reflux ratio of 6.5:1 by volume was employed. The reflux : acetic acid and acetic acid heads ratio were respectively 13:1 and 1:2 by volume.

## Example 2

Distillation was effected continuously in a 45 plate stainless steel column mounted upon a kettle. A feed comprising (% by weight)

52.1% methyl iodide
29.3% methyl acetate
16.7% acetic acid
1.9% acetone

was fed to column at plate 10 at a rate of 2899 ghr$^{-1}$. Acetic acid was fed to the column at plate 40 at a rate of 236 ghr$^{-1}$. The reflux was returned to the head of the column which was maintained at 43°C. At steady state, the overhead and bottom fractions had the following compositions (% by weight - determined by glc).

## Overhead

98.5% methyl iodide
1.0% methyl acetate
0.2% acetic acid
0.3% acetone
Productivity : 545ghr$^{-1}$

## Bottom

37.6% methyl iodide
32.6% methyl acetate
27.8% acetic acid
2.0% acetone
Productivity : 2590 ghr$^{-1}$

A reflux ratio of 7.8:1 by volume was employed. The reflux : acetic acid and acetic acid heads ratios were respectively 8.4:1 and 1:1.1 by volume.

## Claims

1. A process for separating a mixture comprising methyl iodide and methyl acetate which process comprises (a) introducing the mixture of methyl iodide and methyl acetate into the lower half of a distillation zone, (b) introducing acetic acid into the upper half of the distillation zone, (c) removing overhead an overhead fraction having a methyl iodide content greater than the methyl iodide content of a methyl acetate/methyl iodide azeotrope and (d) removing at the bottom of the distillation zone a bottom fraction comprising methyl acetate and acetic acid.

2. A process as claimed in claim 1 wherein the mixture further comprises acetone and the process comprises (a) introducing the mixture of methyl iodide, methyl acetate and acetone into the lower half of a distillation zone, (b) introducing acetic acid into the upper half of the distillation zone, (c) removing overhead an overhead fraction having a methyl iodide greater than the methyl iodide content of a methyl acetate/methyl iodide/acetone azeotrope and (d) removing at the bottom of the distillation zone a bottom fraction comprising methyl acetate, acetone and acetic acid.

3. A process as claimed in either claim 1 or claim 2 wherein the distillation zone is a distillation column having between 20 and 60 plates.

4. A process as claimed in either claim 1 or claim 2 wherein the mixture is introduced into the distillation zone at a point between a third and a half way up from the bottom.

5. A process as claimed in either claim 1 or claim 2 wherein the acetic acid is introduced into the distillation zone at a point two thirds of the way up the column and the top.